(19)

Europäisches
Patentamt

European
Patent Office

Office européen
des brevets

(11) **EP 4 546 360 A1**

(12) **EUROPEAN PATENT APPLICATION**

(43) Date of publication:
**30.04.2025  Bulletin 2025/18**

(21) Application number: **23461673.8**

(22) Date of filing: **27.10.2023**

(51) International Patent Classification (IPC):
**G16H 20/30** $^{(2018.01)}$    **G16H 50/30** $^{(2018.01)}$
**G16H 30/40** $^{(2018.01)}$   **G16H 40/67** $^{(2018.01)}$
**G16H 50/20** $^{(2018.01)}$

(52) Cooperative Patent Classification (CPC):
**G16H 50/30; G16H 20/30;** G16H 30/40;
G16H 40/67; G16H 50/20

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **SHAPE.CARE Sp. z o.o.**
**31-231 Krakow (PL)**

(72) Inventors:
• **KRZYSZKOWSKI, Tomasz**
  **31-207 Krakow (PL)**
• **HOLUBOWICZ, Piotr**
  **31-348 Krakow (PL)**

(74) Representative: **Kancelaria Eupatent.pl Sp. z.o.o**
**Ul. Kilinskiego 185**
**90-348 Lodz (PL)**

(54) **A METHOD AND SYSTEM FOR SUPPORTING PHYSICAL TRAINING EXERCISES**

(57)   A method for supporting physical training exercises, the method comprising: providing a cloud environment (200) comprising an evaluation module (250) with a pre-trained artificial intelligence model (251); receiving (201) at a frontend interface (210) of the cloud environment (200), from a user equipment device (100), a series of user data comprising a user skeleton representation of subsequent image frames representing a user during the performance of the exercise, wherein the user skeleton representation includes information on the position of user body points; analyzing (204) the user data, by means of the machine learning model (251) configured to analyze a particular type of exercise represented by the user data, to generate an assessment related to the performance of the exercise; and outputting (205) feedback information related to the performance of the exercise.

Fig. 1

**Description**

TECHNICAL FIELD

The present invention pertains to methods and systems for supporting physical training exercises, such as physiotherapy or personal training sessions.

BACKGROUND

Physical training, including physiotherapy and personal training, plays a crucial role in maintaining and improving overall health, rehabilitation after injuries, and enhancing athletic performance. These activities often involve a series of physical exercises that need to be performed correctly to achieve the desired results and prevent injuries. However, performing these exercises correctly can be challenging, especially when the individual is not under the direct supervision of a professional trainer or therapist.

Typically, the correctness of the exercise performance is monitored by a professional trainer or physiotherapist, who provides real-time feedback and correction. However, this approach has several limitations. Firstly, it requires the constant presence of a professional, which can be costly and impractical, especially for individuals who prefer or need to train at home. Secondly, even with professional supervision, some subtle mistakes in exercise performance may go unnoticed, especially during complex movements or when the trainer's attention is divided among multiple trainees.

Various tools and devices have been developed to assist in physical training, such as wearable sensors and fitness apps. However, these solutions often only provide quantitative data, such as the number of repetitions or calories burned, and lack the ability to provide qualitative feedback on the correctness of the exercise performance.

SUMMARY

Therefore, there is a need for a system that can monitor the correctness of physical exercise performance in real-time, provide immediate feedback, and suggest improvements, without the constant need for professional supervision. Such a system would greatly enhance the effectiveness and safety of physical training activities, making them more accessible and beneficial for a wider range of individuals.

The method and system according to the invention can support users who perform physical training activities on their own, for example at home, in order to improve their physique or as rehabilitation training. The system can monitor the quantity and quality of performed exercise, detect errors or anomalies and motivate the user to improve the performance.

The object of the invention is a method for supporting physical training exercises, the method comprising: providing a cloud environment comprising an evaluation module with a pre-trained artificial intelligence model; receiving at a frontend interface of the cloud environment, from a user equipment device, a series of user data comprising a user skeleton representation of subsequent image frames representing a user during the performance of the exercise, wherein the user skeleton representation includes information on the position of user body points; analyzing the user data, by means of the machine learning model configured to analyze a particular type of exercise represented by the user data, to generate an assessment related to the performance of the exercise; and outputting feedback information related to the performance of the exercise.

The method may further comprise, at a user equipment device : capturing, by means of a camera module, a video of a user performing the exercise and estimating the pose of the user and generating a representation of the human skeleton.

The feedback information can be output online, during the performance of the exercise.

In one aspect, the machine learning model is pre-trained based on a time series of pre-processed data of reference performers skeleton representations annotated with information about end of a repetition of the exercise and correctness of the repetition; and wherein the machine learning model is configured to further receive user data as a time series and determine whether the time series data represents a correct repetition of the particular exercise. Both the pre-processing of reference performers skeleton representations and the pre-processing of user skeleton representations further comprise centering and rotating the skeleton representations according to the same criteria. The machine learning model is configured to operate based on DTW and KNN methods.

In another aspect, the machine learning model is pre-trained based on pre-processed data of reference performers skeleton representations, wherein the pre-processing comprises generating sets of landmark angles between reference landmark body points, wherein the method comprises pre-processing of user skeleton representations by generating sets of landmark angles between user landmark body points and providing the sets of angles between user landmark body points to the pre-trained machine learning model to determine the pose of the exercise performed by the user. The machine learning model is XGBoost, RandomForestClassifier, KNN or SVM. The method further comprises analyzing data corresponding to subsequent frames of user recording, by determining a pose of the user in a particular frame and upon analyzing a plurality of frames, determining whether subsequent poses form a predetermined series of poses corresponding to a particular exercise and if so, determining the set as corresponding to a complete repetition of the exercise.

The invention also relates to a computer-implemented system comprising at least one non-transitory processor-readable storage medium that stores at least one of processor-executable instructions or data and at least one processor communicably coupled to at least one non-transitory processor-readable storage medium and configured to perform the steps of the method as described herein.

BRIEF DESCRIPTION OF DRAWINGS

Various embodiments are herein described, by way of example only, with reference to the accompanying drawings, wherein:

Fig. 1 shows an overall diagram of the system.
Fig. 2 presents an example of a set of user body points;
Fig. 3 illustrates a diagram of functional modules;
Figs. 4a, 4b, 4c show examples of frames representing a performer;
Fig. 5 presents an example of a data pre-processing alignment procedure;
Figs. 6a and 6b show a skeleton in a position before a rotation and after a shift and rotation, correspondingly;
Figs. 7a and 7b illustrate a procedure of forming clusters;
Fig. 8 shows a procedure utilizing a machine learning model based on clusters of body points;
Fig. 9 shows an example of input reference data;
Fig. 10 shows an example of a set of angles;
Fig. 11 shows a procedure utilizing a machine learning model based on angles between landmark points;
Fig. 12 shows an overall procedure for recommending a training plan;
Fig. 13 presents a computer-implemented system.

DETAILED DESCRIPTION

The following detailed description is of the best currently contemplated modes of carrying out the invention. The description is not to be taken in a limiting sense but is made merely for the purpose of illustrating the general principles of the invention.

Overview of the method and system

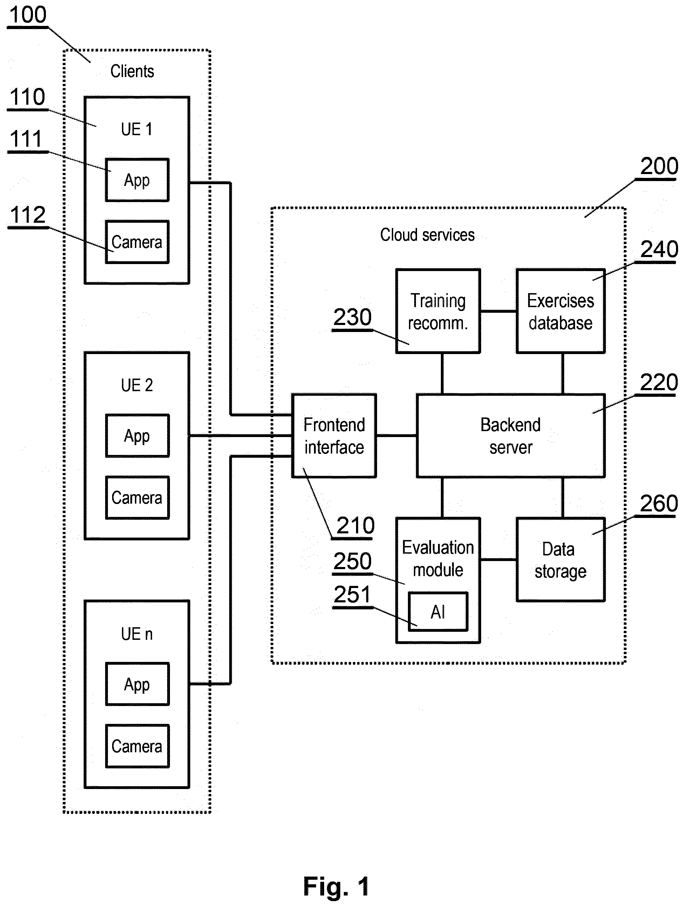

The system as shown in Fig. 1 comprises a client part 100 and a cloud environment 200. The client part includes a plurality of user equipment (UE) devices 110, such as mobile devices, such as smartphones, operating a dedicated software application (App) 111. The software application 111 captures image data and performs initial analysis.

The cloud environment 200 comprises a frontend interface module 210 configured to communicate with the client software applications 111 to collect data and balance traffic. The collected data is input to a backend server module 220 that is configured to coordinate the operation of the system. A training recommender module 230 is connected with a training database 240 that contains data related to various types of exercises and their cluster data. The training recommender module 230 is configured to recommend and adapt training plans for users. An evaluation module 250 is configured to process data received from users, segment data and assess whether the training is performed correctly, based on an at least one artificial intelligence model 251 that operates a machine learning model, and to provide feedback for the user. The user training data and reference data are stored in data storage module 260.

The artificial intelligence model 251 comprises at least one machine learning model that is trained to assess the data collected from a user and to assess whether the user performs a particular exercise in a correct manner. For example, for each exercise type, a dedicated machine learning model can be used, being trained with data corresponding to that exercise. It has been determined that for initial training, data collected from at least 50 persons, each performing between 5 and 20 repetitions of a particular exercise, is sufficient to allow the system to process data for assessment of other users. Preferably, the recordings are done as would be done by the users of the system, for example with a camera positioned about 2-3 meters from the user. Preferably, the users should be of diverse age and sex, and should perform the exercises in various surroundings and lighting conditions. However, after the system is set up, the machine learning models can be further trained by data collected from other users, which have been qualified (by a skilled physician) as related to a correctly performed exercise.

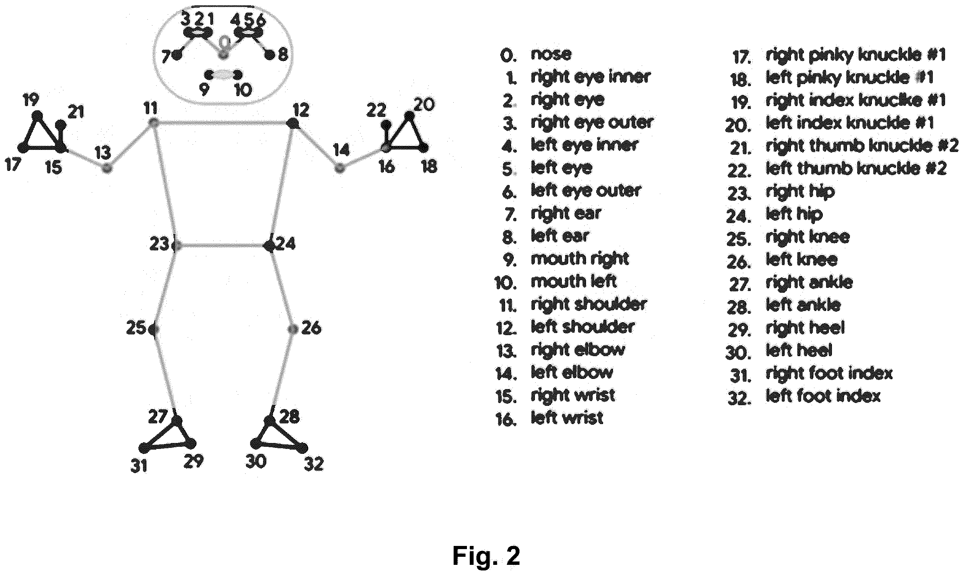

In order to process the data, the system is configured to detect a number of points on the body of the user. Depending on the exercise performed, the system can use a subset of particular landmark points (which can be all body points or body points most suitable for that particular exercise) that have a crucial importance for the assessment of that particular exercise. Fig. 2 presents an example of a set of user body points.

In order to reduce the amount of data transmitted between the user equipment 110 and the frontend interface 210, the software application 111 at the user equipment is configured to perform pre-processing of captured video data in order to detect the user body landmark points and to transmit to the frontend interface 210 only descriptors of movement of landmark points instead of full video stream. This has the additional advantage that it protects the user's privacy, as no user image is transmitted to the cloud environment.

The body points can be detected using known methods, such as by using a MediaPipe Pose Landmarker with a BlazePose model, and its function HumanPoseEstimation. The inventors found that it is efficient to first determine landmarks in the video captured by the camera, and then divide the video by tags.

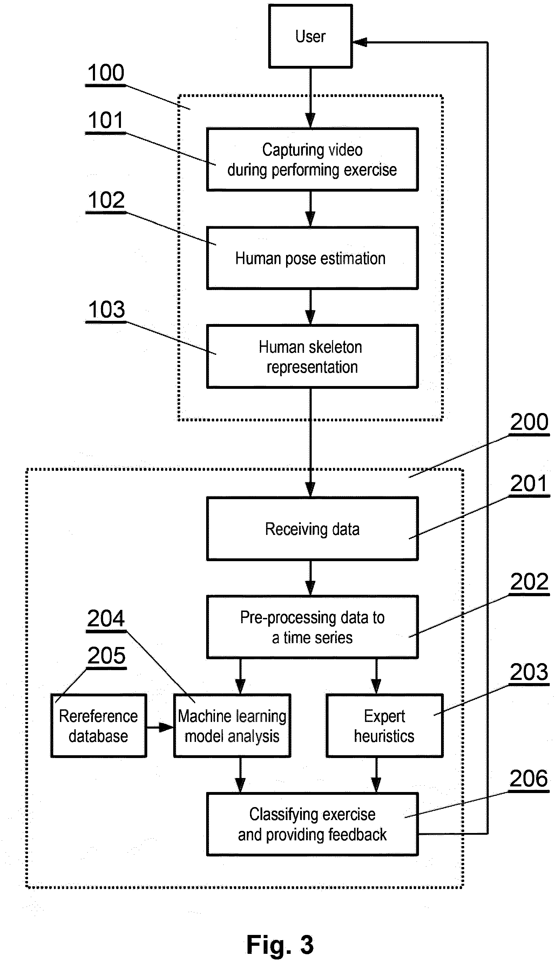

Fig. 3 illustrates a diagram of functional modules and steps utilized at the user equipment device 110 (and managed by the application 111) and at the cloud environment 200.

The system operates as follows: The user initiates the software application 111 at the user equipment 100. Through the application 111, the user selects a desired training module containing a set of exercises to be performed. A training plan may be presented to the user, including information such as a list of exercises, a description of each exercise, videos demonstrating model performance of the exercise, and a suggested training plan with information about the number of sets of each exercise, number of repetitions within each set, amount of weight (related to weight lifting exercises), etc., tailored to the specific user. The user initiates the start of the training. The application instructs the user on how to position the user equipment to properly capture the user's image via the camera 112 in module 101. Subsequently, while the user performs the exercise, the application 111 conducts pre-processing of the video data captured by the camera 112 in a human pose estimation module 102 to determine the user's pose, as a set of landmark points (selected from the full set of body points shown in Fig. 2), in successive video frames, depending on the desired time frequency (at least 60fps). Next, a data set containing the user poses during the performance of the exercise is prepared in the human skeleton representation module 103. The data set, corresponding to the representation of the skeleton on one or more video frames, is sent to the cloud environment 200, where it is received by a data pre-processing module that transforms them into a time series, by combining skeleton data from one or more video frames (online as received from the application or upon collecting a required number of frames). Subsequently, the transformed time series is input by module 202 into a machine learning model 204 (which forms a part of the artificial intelligence module 251 of the evaluation module 250) that is trained with reference data 205 (which is stored in a data storage 260). The time series is also input to an expert heuristics module 203 for providing training data to the machine learning module. Finally, the exercise is classified with respect to the correctness of its performance and feedback is generated in module 206, and the feedback is sent back to the user, preferably online during the performance of the exercise, so that the user can correct the way in which the exercise is performed. Depending on the selected embodiment of evaluation procedure, the assessment may include information about whether exercise is performed correctly, whether all body landmark points are visible and how many correct and incorrect repetitions of the exercise have been performed (for the first embodiment described below) or also a more advanced assessment, such as a detailed assessment of the performance of particular poses of the exercise, which angles based on landmark points are away from the expected position and by how much (for the second embodiment described below).

One of the characterizing features of the present invention is the machine learning models used to analyze the exercises performed by the users. Two types of characteristic machine learning models can be used, as will be explained in detail below.

First embodiment - a procedure utilizing a machine learning model based on DTW and KNN

The machine learning model according to the first embodiment is designed to analyze time series of frames including information on position of body points and detecting which repetitions of the exercise were performed correctly.

The data needs to be pre-processed first in order to rotate and center the skeleton to a reference position. For example, it can be a position defined as "head facing north, belt buckle in the center of the coordinate system".

Training data for the machine learning model is provided to the system as a multitude of training data sets, wherein each data set is identified by a performing actor (performer), exercise identifier, and repetition identifier (a multitude of repetitions can be provided for a single exercise performed by the same performer), information about the timing of the beginning and ending of the performance of a single repetition (can be provided as a separate file with timing tags), as well as an indication of whether the particular exercise repetition was performed correctly.

The quality metrics of performance are determined using a leave-one-performer-out cross-validation method. Sampling of items into the test set in cross-validation is done at the performer identifier level, not the repetition identifier, since one person typically performs all repetitions in a way specific to that person. Thus, the machine learning model is validated by a "random performer", i.e., one whose repetitions have not been used for training the model. The training set is also called a reference set because of the "information-based" nature of the selected model, i.e.: without reference to references, the model is unable to make a classification decision.

For the purposes of training the models, data annotated by an expert is used. The test data are annotated by an expert to segment the performed exercises into repetitions, i.e., the recording of a person performing a series of repetitions is divided into individual repetitions. This element of recording segmentation is common to both methods and helps to protect the appropriate set of frames for further analysis, regardless of whether the DTW and KNN method of the present embodiment or the method of the angles according to the second embodiment described, which is described later. In a simple embodiment, this can be done manually.

An example tag file may have the following structure:

{"peaks.point.1":{"startTime":19.934126,"id":"peaks.point.1","labelText":"start"},"peaks.poi          nt.2":{"startTime":21.24959,"id":"peaks.point.2","labelText":"ic"},"peaks.point.3":{"startTi   me":23.266627,"id":"peaks.point.3","label-Text":"c"},...

"Peaks point" refers to a point in time. It can be labeled as "start" denoting starting of a series of exercises, "c" denoting a finished correctly performed repetition or "ic" denoting an incorrectly performed repetition.

The data processed by the system is automatically segmented each time. The number of repetitions of a particular exercise can be based on the classification of a pose. For each exercise, it is necessary to define poses, which can be classified in a sequence defining the whole performance of the exercise. The machine learning model can provide a prediction of which particular pose is presented on a particular frame. If the poses occur in a predefined sequence, then that full sequence can be classified as a repetition of the exercise.

Next, the machine learning model is trained based on the time series data, based on a method combining a DTW method (to analyze data in time series) and KNN method (to determine similarity between user data and reference data).

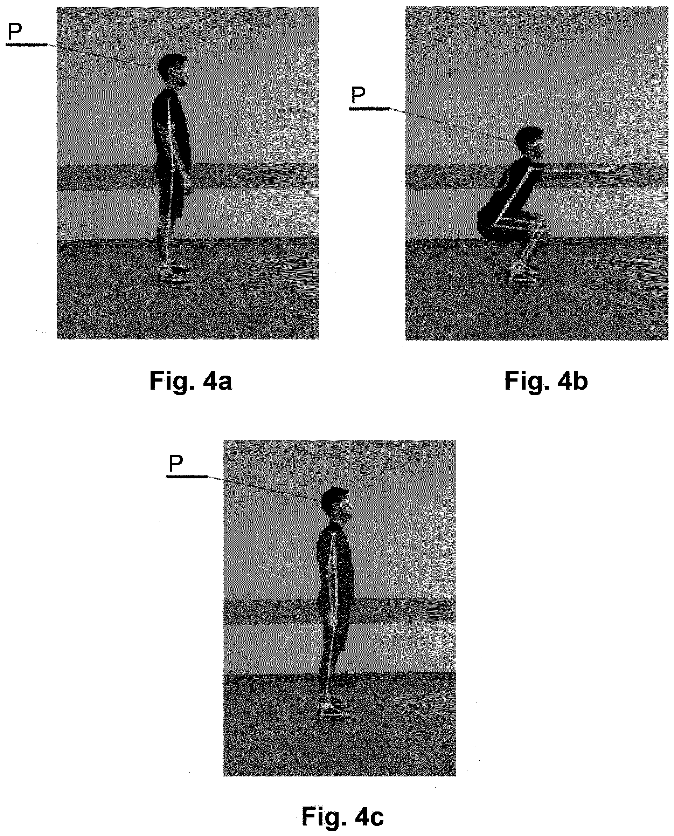

Figs. 4a, 4b, 4c show examples of frames representing a performer in an upright state (Figs. 4a, 4c) and a squat state (Fig. 4b), respectively, with visible landmark points P.

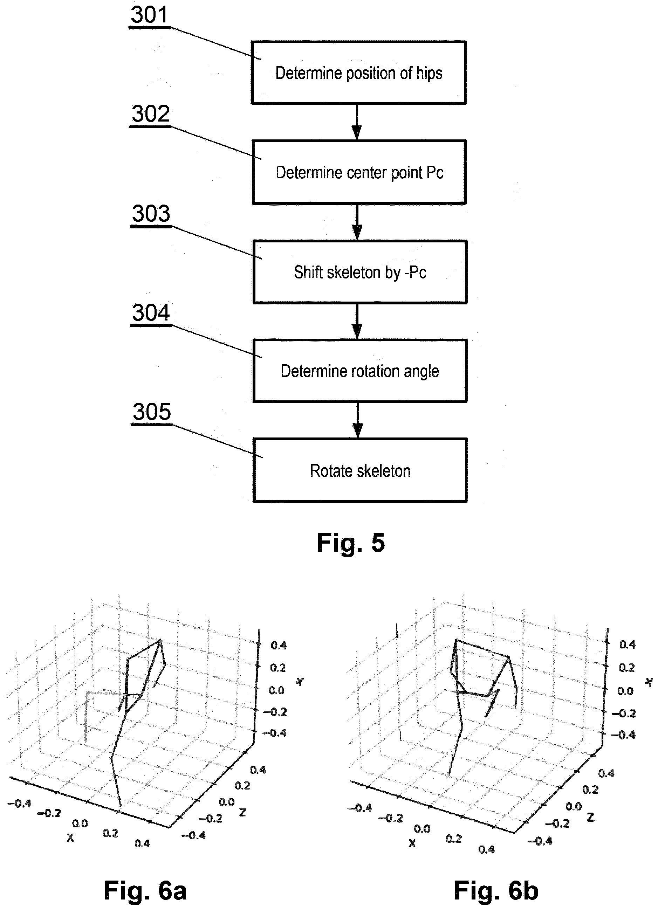

Before a procedure utilizing a machine learning model based on DTW and KNN can be used, the data has to be pre-processed to align the skeleton body points with respect to a coordinate system in the same manner as in the reference data. This is initially achieved by instructing the user to take a specific position with respect to the camera. Next, the detected skeleton data can be further aligned in detail. For example, a data pre-processing alignment procedure may be performed as specified in Fig. 5. The first step 301 includes determining the position of the hip body points. Next, in step 302, coordinates of a center point Pc are determined in a segment that connects the right and left hip. In step 303 the entire skeleton is shifted by coordinates of -Pc, to have that central point located in the center of the coordinate system. Next, in step 304 an angle of rotation of the line connecting the hips with respect to the OX axis is determined. In step 305 the entire skeleton is rotated by that angle of rotation with respect to the OX axis, by multiplying the coordinate matrix of the skeleton by the rotation matrix by the determined angle, such that finally the hip bone is parallel to the OX axis. Figs. 6a and 6b show the skeleton in a position before the rotation and the skeleton after the shift and rotation, respectively.

The angle of rotation of the hip bone can be determined using an atan2 algorithm, since it could not be done using simple trigonometric functions. For angles in the interval [0,360), this algorithm returns a unique value in the interval $(-\pi,\pi)$. Rotating the skeleton ignores that the hip bones have z-coordinates. The atan2 algorithm is summarized as follows: Atan2:

$$x, y \in \mathbb{R}^2$$

$$\mathrm{atan2}(x,y) = \begin{cases} \arctan(\frac{y}{x}) & \text{if } x > 0 \\ \arctan(\frac{y}{x}) + \pi & \text{if } x < 0 \text{ and } y \leq 0 \\ \arctan(\frac{y}{x}) - \pi & \text{if } x < 0 \text{ and } y < 0 \\ +\frac{\pi}{2} & \text{if } x = 0 \text{ and } y > 0 \\ -\frac{\pi}{2} & \text{if } x = 0 \text{ and } y < 0 \\ \text{undefined} & \text{if } x = 0 \text{ and } y = 0 \end{cases}$$

"DTW one vs. DTW all" algorithm requires that each of the series of the reference set has the same length, and that each element of the row is responsible for the same part (pose) of the exercise. For example, the upmost position of the "jumping jack" exercise in each of the series of the reference set should have the same pose index. In the case of unnormalized data, the "DTW one vs DTW all algorithm" could match elements non-chronologically, i.e., a pose of exercise A could be considered as another pose of exercise B, or pose A would not be matched with pose A from another row because of the shift of exercise parts relative to each other.

The inventors found that the procedure can be effectively performed using a machine learning model trained on data sets represented as clusters by a "K-medoids" algorithm, which finds k clusters in a data set. It minimizes the distance between points assigned to the same cluster and the point designated as the cluster center (the cluster center is also called the centroid). Furthermore, the algorithm selects actual points from the data set as centroids. This allows for more interpretable clustering and the use of metrics other than Euclidean (such as the DTW metrics used herein).

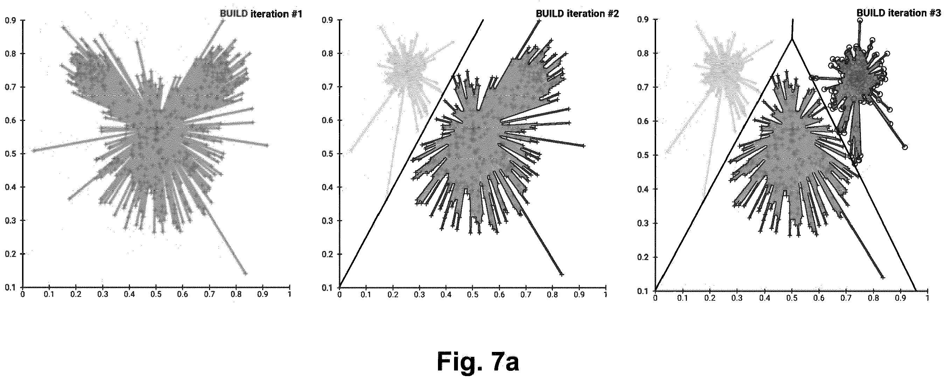

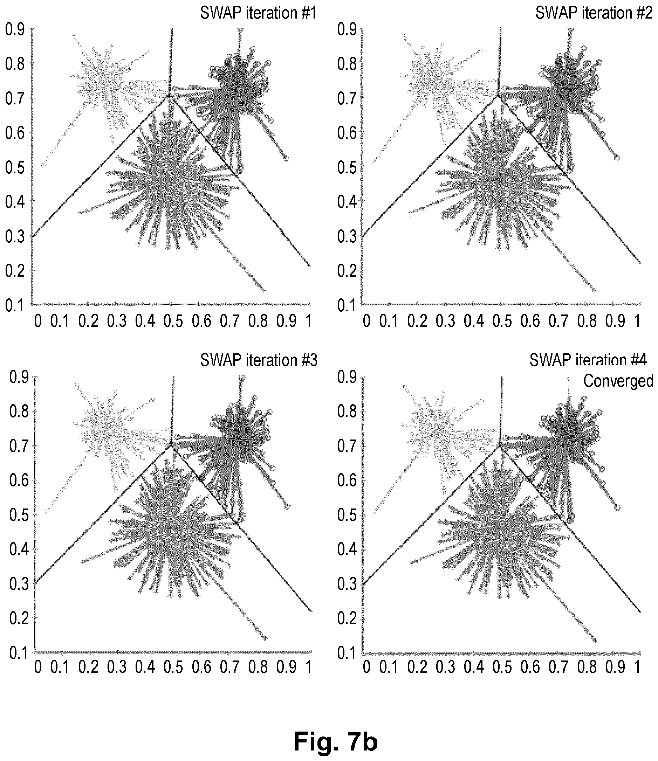

The procedure of forming clusters is illustrated in Figs. 7a and 7b. Fig. 7a shows three iterative steps of building three clusters. Fig. 7b shows subsequent iterative steps of swapping clusters and determining centers and points belonging to the actual data set. A point is considered as belonging to a cluster if the distance of this point to the centroid of this cluster is less than the distance to the centroids of the other clusters. Determining clusters for given centroids involves matching each point from the data set to its nearest centroid. Therefore, the procedure for building clusters receives, as input, a set of data and a number k of centroids to be searched for, and outputs data corresponding to k clusters.

The K-medoids algorithm is non-deterministic and can return different centroids each time it is executed, if k>1. Therefore, it should be executed several times, until it is possible to find the most frequently recurring elements as the mean.

The clusters formed in this manner represent a data series corresponding to a particular exercise performed by different reference performers. These data are stored within the machine learning model once it is trained based on the reference time series.

Next, the trained machine learning model 251 is utilized to predict whether the user performed the exercise correctly by comparing a time series representing user data and converted to clusters with centroids with the pre-trained clusters and centroids of the reference data, by means of k-nearest neighbor (KNN) algorithm.

The KNN algorithm calculates the distances of an element to be classified to each of the elements of the reference data. The KNN algorithm provides, as an output, an identifier of the reference data element that contains centroids to which the centroids of the currently analyzed data were closest, along with a score. Based on this, a probability that the currently analyzed data corresponds to a specific correctly performed exercise repetition can be determined.

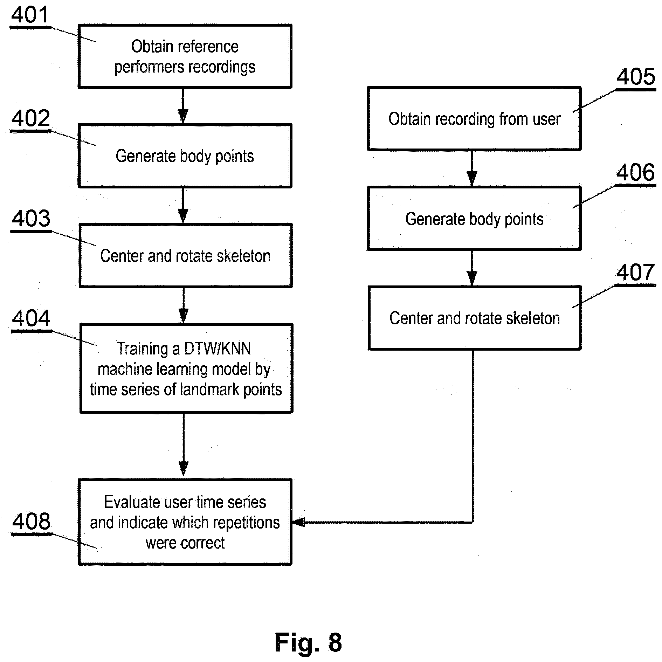

The procedure explained above is summarized in Fig. 8. First, in step 401, recordings of reference performers (actors) who perform a specific exercise are obtained. The recordings are annotated by experts to generate tags indicating start of exercise, ends of individual repetitions and indication on whether the repetition was performed correctly or incorrectly. Next, landmark points for these recordings are generated in step 402. Then, the reference data are pre-processed by centering and rotating in step 403. In step 404, the pre-processed time series of a reference exercise is input to the DTW/KNN machine learning model 251 to train the model to recognize the particular exercise, wherein the machine learning model 251 generates clusters with centroids as its internal data. This completes the step of preparing the environment for classification of data provided from users. Steps 401-404 are performed at the cloud environment side. Next, in step 405, as a user performs the exercise, the video data is collected via the camera 112. In step 406, data is pre-processed by the software application 111 by generating landmark points for successive video frames (with a predefined frequency, preferably at least 60 fps). In step 407 the landmark data are further pre-processed by centering and rotating. At least steps 405-406 are performed at the user equipment 100 and step 407 can be performed either at the user equipment device 100 or at the cloud environment 200. In step 408, the time series representing user data is input to the pre-trained machine learning model configured to determine, using the DTW and KNN algorithms based on the reference clusters data, a probability that the specific user time series data corresponds to a correct or incorrect repetition of the exercise. Data can be provided in a continuous manner, so that the model can provide a live output indicating whether the recently processed data contained a correct or incorrect repetition of the exercise.

Second embodiment - a procedure utilizing a machine learning model based on angles between landmark points

This embodiment is similar to the first embodiment with respect to the initial steps related to determining landmark points on subsequent frames during performance of the exercise.

Once the landmark points are defined, specific landmark angles are determined between predefined (specific for a particular exercise, and also preferably specific for a particular pose) landmark points. Therefore, this embodiment searches for frames in which the angle values form the best match with reference angles in specific poses. For each characteristic pose, only a single frame that most accurately represents the expected angle values is selected as a reference. This procedure allows for the extraction of key moments of an exercise from the sequence that are important for evaluating the correctness of the exercises performed.

A pose is defined as a set of angles between lines connecting body landmark points relevant for that exercise. To each angle a threshold value can be defined, indicating allowable limits within which that angle can vary. For each frame data is stored that defines all the angle values for connections of landmark points (not only those defined at a given pose), as well as the identifier of the defined pose.

The poses are defined for each exercise individually. A pose is defined as a set of angles between lines connecting body landmark points relevant for that exercise. To each angle a threshold value can be defined, indicating allowable limits within which that angle can vary. Single repetitions are extracted from reference training data and for each repetition a sequence of all frames is analyzed. For each frame, landmark body points are defined along with angles between lines connecting these points. For example, a particular exercise may have a set of 14 the following landmark points defined: right_knee, left_knee, right_foot, left_foot, right_leg, left_leg, right_arm-collarbone, left_arm-collarbone, right_arm-hip, left_arm-hip, right_elbow, left_elbow, right_hip, left_hip.

For each frame data is stored that defines all the angle values for connections of landmark points (not only those defined at a given pose), as well as the identifier of the defined pose.

The following example pertains to a type of exercise: squats. Two states of the exercise are defined:

- a squat pose (class 0), i.e., a pose where the angle at the knee (hip-knee-ankle) should be 90 degrees, and the angle of the leg (shoulder-hip-knee) should be less than 90 degrees;

- an upright pose (class 1), i.e., a pose where the angle at the knee (hip-knee-ankle) should be 180 degrees, and the angle of the leg (shoulder-hip-knee) should be 180 degrees.

A threshold value for both conditions can be set as 30 degrees, i.e., all frames in the reference repetition that meet the conditions for the 1st and 2nd pose respectively with the possibility of deviation of 30 degrees will be analyzed, but data for the training set will be added only for the frame that is closest to the angles defined for the poses.

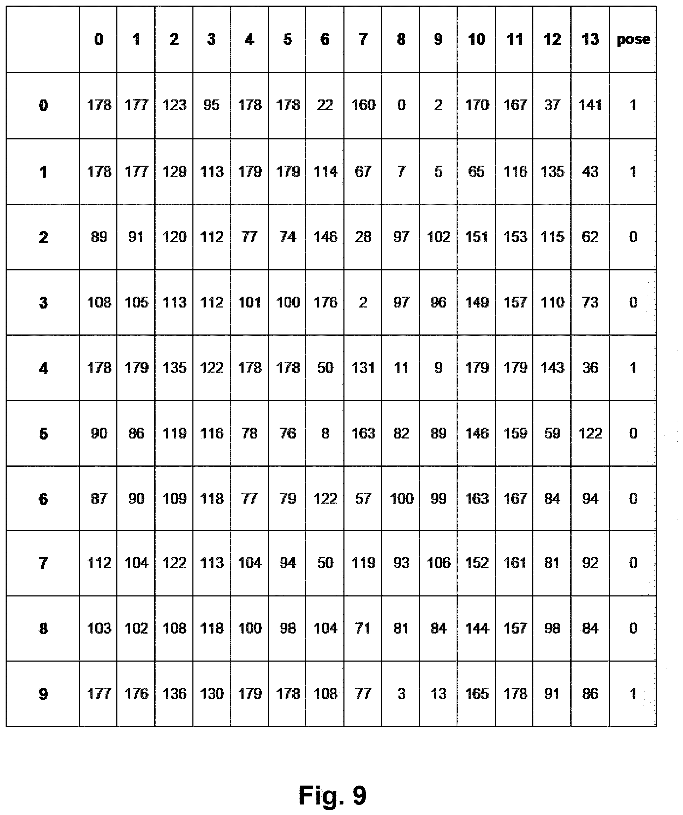

For instance, the input reference data shown in Fig. 9 may contain frames labeled 0-9 (represented by rows), where for each frame, values of angles are presented in columns 0-13 and a pose class (0 - squat, 1-upright) is indicated in the last column. Columns 0-13 represent the following set of angles: 0 - right_knee, 1 - left_knee, 2 - right_foot, 3- left_foot, 4 - right_leg, 5 - left_leg, 6 - right arm-collarbone, 7 - left arm-collarbone, 8 - right arm-hip, 9 - left_arm-hip, 10 - right elbow, 11 - left elbow, 12 - right_hip, 13 - left_hip.

A plurality of extracted angles are combined to a set of landmark angles forming a data representation for a single repetition of the exercise. For example, if the exercise comprises two characteristic poses and each pose is represented by fourteen angles, the reference landmark angles dataset comprises a total of 28 landmark angle values - 14 angles for pose 0 and 14 angles for pose 1. This tabular dataset is the basis for further analysis.

To select the optimal parameters, a cross-validation technique can be used, preferably a Leave One Out Cross-Validation (CV). This process involves iteratively training the machine learning model on all repetitions, excluding those performed by a specific individual. Then, the left-out repetitions are used to test the quality of the prediction. This strategy allows the overall performance of the model to be evaluated on data that was not used in the training process. Metrics are calculated from the aggregated results for all individuals performing the exercises. Machine learning models that were found as particularly preferable in this step are: XGBoost, RandomForestClassifier, KNN, SVM.

The machine learning model for evaluating exercise correctness can be trained based on the values of the angles in the characteristic reference poses. Due to the high complexity of these models, it is important to indicate what characteristics influenced the exercise to be considered correct or incorrect. A contribution of each landmark angle value to the prediction can be determined using Shapley values, specifically SHAP values as explained in an article "A unified approach to interpreting model predictions" (by Lundberg, Scott M., and Su-In Lee in Advances in neural information processing systems 30 (2017)).

The goal of the method is to explain the result of the model (prediction) for a selected repetition of the exercise by calculating the contribution of each feature to the prediction. The result of the model can be explained by assuming that each instance trait value is a "player" in a game where the prediction is the payoff. The Shapley value explains how to fairly distribute the "payoff" among the traits. A detailed theoretical description can be found, among others, in a publication "Explanatory model analysis: explore, explain, and examine predictive models" (by Biecek, Przemyslaw, and Tomasz Burzykowski, CRC Press, 2021).

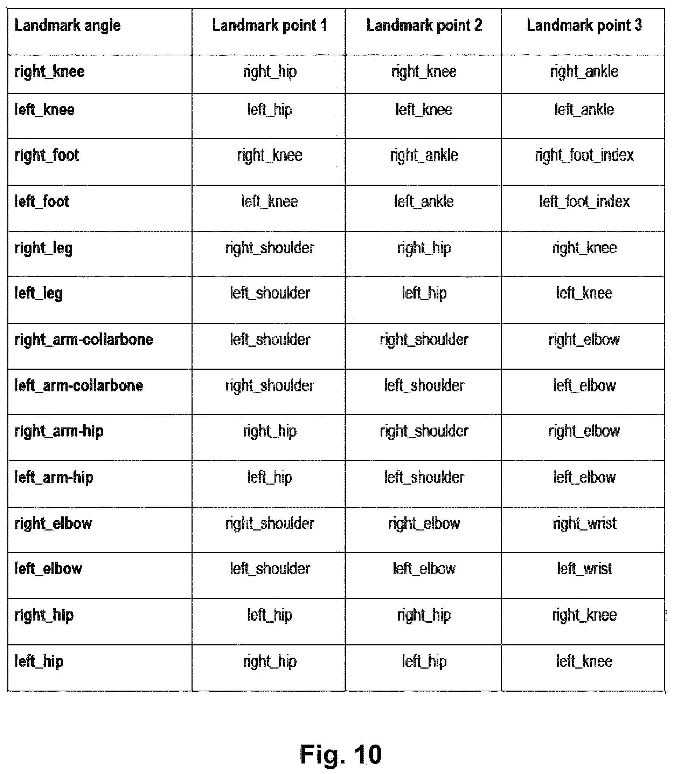

For example, a set of 14 landmark angles, each defined between 3 landmark points of the body, as shown in Fig. 10, is sufficient to determine most of the exercise types to be evaluated.

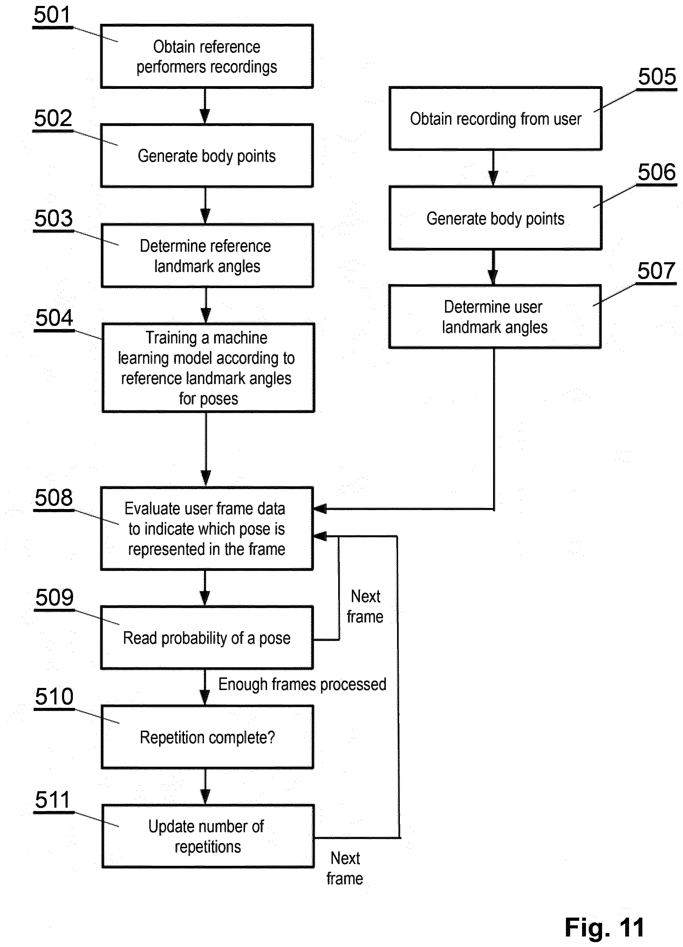

The procedure explained above is summarized in Fig. 11. Its beginning is substantially equivalent to the procedure of Fig. 8, wherein steps 501, 502, 505, 506 correspond to steps 401, 402, 405, 406 respectively. In steps 503 and 507 landmark angles are determined for reference and user poses. Since the landmark angles are independent of the position of the skeleton in the coordinate system, it is not necessary to perform steps equivalent to 403 and 507, i.e., it is not necessary to shift and rotate the skeleton to the same position. In step 504, sets of reference landmark angles along with annotated poses of particular exercise are input to the machine learning model 251, such as XGBoost, RandomForestClassifier, KNN or SVM, to train the model to recognize the particular poses based on landmark angles. In step 508 the pre-processed user data corresponding to angles on consecutive frames is input to the machine learning model 251 configured to determine a probability that the specific data corresponds to a specific pose of the exercise. In step 509, if the probability is higher than a specific threshold, the evaluation module considers that a specific state (pose) of the exercise has been achieved in the analyzed frame. A subsequent frame is analyzed next. Once a number of frames have been analyzed, the evaluation module checks in step 510 whether all the predetermined poses corresponding to the specific type of exercise have been achieved in a correct order and if so, the analyzed frames are considered as representing a repetition of the exercise and the counter of repetitions is increased in step 511. The procedure continues to examine further frames. In order to make the procedure immune to incidental, erroneous and noisy data, it is possible to define a minimum duration of a single repetition, so that potentially erroneous predictions close to the defined threshold do not erroneously increase the number of repetitions.

Recommendations for training plan

When creating a training plan, load grading should be taken into account, particularly for rehabilitation purposes. Load grading includes the selection of an appropriate number of sets and repetitions within a set, the duration of intervals between sets, training intensity, frequency, and duration of workouts.

Exercises should be planned so that a particular body part or muscle is not overstimulated. The exercises and their intensity should be selected individually according to the capabilities of the patient. The proposed recommendations should be approved by a supervisor each time, provided that the patient feels fine and increasing the training intensity is possible until it is not dangerous for the patient.

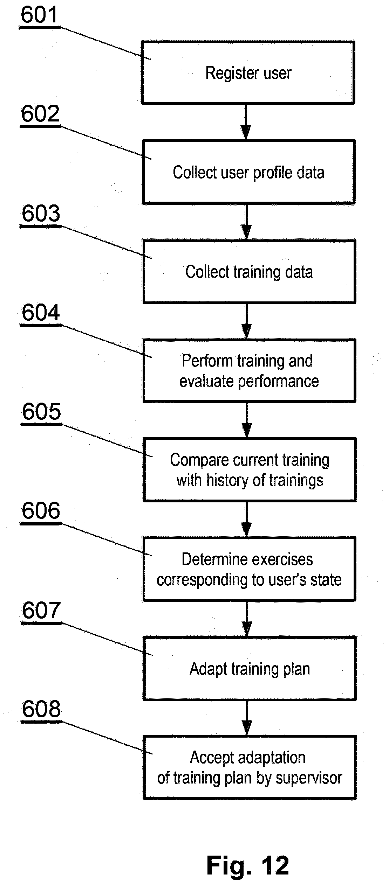

Fig. 12 shows an overall procedure for recommending a training plan. In step 601, the user registers in the system. This involves the user installing the dedicated software application 111 on the user equipment device 100, such as a smartphone. The user also provides health data to the cloud environment 200. An initial training plan is prepared for the user, typically by a skilled supervisor. In step 602, the user initiates a training session and the user profile is read at the cloud environment 200. Next, training data is collected that indicates a set of exercises to be performed by the user during the current training session. In step 604, the user performs the training. While the user performs the training, the user device 100 captures a video of the user via the camera 112, pre-processes the video data to determine landmark points by the software application 111, sends the pre-processed data to the cloud environment 200, where the backend server 220 instructs the evaluation module 250 to use the artificial intelligence module 251 to evaluate the performance of the exercise. The evaluation may include information on which exercises were performed correctly and to what extent (e.g. percentage of correct repetitions). In step 605 the evaluation is received by the training recommendation module 230, which compares it with a history of past performances. In step 606, the training recommendation module 230 determines exercises that are appropriate for the particular user (depending on the user profile data, e.g. information on which skills are to be trained or what kind of illness is to be treated), and next in step 607 adapts the training plan to match it to the user's needs and performance. The adapted training plan should be reviewed in step 608 by the skilled supervisor and is stored for future use to determine the exercises to be performed in the next training session.

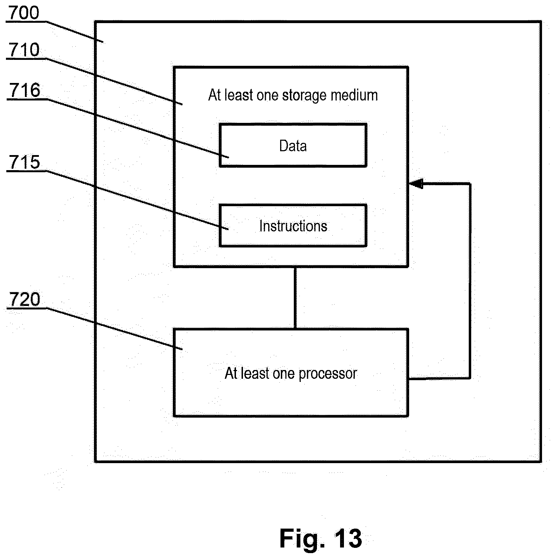

The functionality of the cloud environment described herein can be implemented, for example, in a computer-implemented system 700, such as shown in Fig. 13. The system may include at least one non-transitory processor-readable storage medium 710 that stores at least one of processor-executable instructions 715 or data 716 and at least one processor 720 communicably coupled to at least one non-transitory processor-readable storage medium. At least one processor is configured to perform the steps of the methods presented herein to implement the functionality of the modules of the cloud environment 200 presented in Fig. 1.

While the invention has been described with respect to a limited number of embodiments, it will be appreciated that many variations, modifications and other applications of the invention may be made. Therefore, the claimed invention as recited in the claims that follow is not limited to the embodiments described herein.

**Claims**

1.    A method for supporting physical training exercises, the method comprising:

      - providing a cloud environment (200) comprising an evaluation module (250) with a pre-trained artificial intelligence model (251);
      - receiving (201) at a frontend interface (210) of the cloud environment (200), from a user equipment device (100), a series of user data comprising a user skeleton representation of subsequent image frames representing a user during the performance of the exercise, wherein the user skeleton representation includes information on the position of user body points;
      - analyzing (204) the user data, by means of the machine learning model (251) configured to analyze a particular type of exercise represented by the user data, to generate an assessment related to the performance of the exercise; and
      - outputting (205) feedback information related to the performance of the exercise.

2.    The method according to claim 1, further comprising, at a user equipment device (100):

      - capturing (101), by means of a camera module (112), a video of a user performing the exercise,

- estimating (102) the pose of the user and generating (103) a representation of the human skeleton.

3.  The method according to any of the previous claims, wherein the feedback information is output (205) online, during the performance of the exercise.

4.  The method according to any of the previous claims, wherein the machine learning model (251) is pre-trained based on a time series of pre-processed data of reference performers skeleton representations annotated with information about end of a repetition of the exercise and correctness of the repetition; and wherein the machine learning model (251) is configured to further receive user data as a time series and determine whether the time series data represents a correct repetition of the particular exercise.

5.  The method according to claim 4, wherein both the pre-processing of reference performers skeleton representations and the pre-processing of user skeleton representations further comprise centering and rotating the skeleton representations (403, 407) according to the same criteria.

6.  The method according to any of claims 4-5, wherein the machine learning model (251) is configured to operate based on DTW and KNN methods.

7.  The method according to any of claims 1-3, wherein the machine learning model (251) is pre-trained based on pre-processed data of reference performers skeleton representations, wherein the pre-processing comprises generating (503) sets of landmark angles between reference landmark body points, wherein the method comprises pre-processing of user skeleton representations by generating (507) sets of landmark angles between user landmark body points and providing (508) the sets of angles between user landmark body points to the pre-trained machine learning model (251) to determine the pose of the exercise performed by the user.

8.  The method according to claim 7, wherein the machine learning model (251) is XGBoost, RandomForestClassifier, KNN or SVM.

9.  The method according to any of claims 7-8, further comprising analyzing data corresponding to subsequent frames of user recording, by determining (508) a pose of the user in a particular frame and upon analyzing a plurality of frames, determining whether subsequent poses form a predetermined series of poses corresponding to a particular exercise and if so, determining (510) the set as corresponding to a complete repetition of the exercise.

10. A computer-implemented system (700) comprising at least one non-transitory processor-readable storage medium (710) that stores at least one of processor-executable instructions (715) or data (716) and at least one processor (720) communicably coupled to at least one non-transitory processor-readable storage medium (710) and configured to perform the steps of the method according to any of previous claims.

**Fig. 1**

0. nose
1. right eye inner
2. right eye
3. right eye outer
4. left eye inner
5. left eye
6. left eye outer
7. right ear
8. left ear
9. mouth right
10. mouth left
11. right shoulder
12. left shoulder
13. right elbow
14. left elbow
15. right wrist
16. left wrist
17. right pinky knuckle #1
18. left pinky knuckle #1
19. right index knuckle #1
20. left index knuckle #1
21. right thumb knuckle #2
22. left thumb knuckle #2
23. right hip
24. left hip
25. right knee
26. left knee
27. right ankle
28. left ankle
29. right heel
30. left heel
31. right foot index
32. left foot index

Fig. 2

**Fig. 3**

Fig. 4a

Fig. 4b

Fig. 4c

301

Determine position of hips

302

Determine center point Pc

303

Shift skeleton by -Pc

304

Determine rotation angle

305

Rotate skeleton

**Fig. 5**

**Fig. 6a**

**Fig. 6b**

**Fig. 7a**

Fig. 7b

401
Obtain reference
performers recordings

402
Generate body points

403
Center and rotate skeleton

404
Training a DTW/KNN
machine learning model by
time series of landmark points

405
Obtain recording from user

406
Generate body points

407
Center and rotate skeleton

408
Evaluate user time series
and indicate which repetitions
were correct

**Fig. 8**

|   | 0 | 1 | 2 | 3 | 4 | 5 | 6 | 7 | 8 | 9 | 10 | 11 | 12 | 13 | pose |
|---|---|---|---|---|---|---|---|---|---|---|----|----|----|----|------|
| 0 | 178 | 177 | 123 | 95 | 178 | 178 | 22 | 160 | 0 | 2 | 170 | 167 | 37 | 141 | 1 |
| 1 | 178 | 177 | 129 | 113 | 179 | 179 | 114 | 67 | 7 | 5 | 65 | 116 | 135 | 43 | 1 |
| 2 | 89 | 91 | 120 | 112 | 77 | 74 | 146 | 28 | 97 | 102 | 151 | 153 | 115 | 62 | 0 |
| 3 | 108 | 105 | 113 | 112 | 101 | 100 | 176 | 2 | 97 | 96 | 149 | 157 | 110 | 73 | 0 |
| 4 | 178 | 179 | 135 | 122 | 178 | 178 | 50 | 131 | 11 | 9 | 179 | 179 | 143 | 36 | 1 |
| 5 | 90 | 86 | 119 | 116 | 78 | 76 | 8 | 163 | 82 | 89 | 146 | 159 | 59 | 122 | 0 |
| 6 | 87 | 90 | 109 | 118 | 77 | 79 | 122 | 57 | 100 | 99 | 163 | 167 | 84 | 94 | 0 |
| 7 | 112 | 104 | 122 | 113 | 104 | 94 | 50 | 119 | 93 | 106 | 152 | 161 | 81 | 92 | 0 |
| 8 | 103 | 102 | 108 | 118 | 100 | 98 | 104 | 71 | 81 | 84 | 144 | 157 | 98 | 84 | 0 |
| 9 | 177 | 176 | 136 | 130 | 179 | 178 | 108 | 77 | 3 | 13 | 165 | 178 | 91 | 86 | 1 |

# Fig. 9

| Landmark angle | Landmark point 1 | Landmark point 2 | Landmark point 3 |
|---|---|---|---|
| right_knee | right_hip | right_knee | right_ankle |
| left_knee | left_hip | left_knee | left_ankle |
| right_foot | right_knee | right_ankle | right_foot_index |
| left_foot | left_knee | left_ankle | left_foot_index |
| right_leg | right_shoulder | right_hip | right_knee |
| left_leg | left_shoulder | left_hip | left_knee |
| right_arm-collarbone | left_shoulder | right_shoulder | right_elbow |
| left_arm-collarbone | right_shoulder | left_shoulder | left_elbow |
| right_arm-hip | right_hip | right_shoulder | right_elbow |
| left_arm-hip | left_hip | left_shoulder | left_elbow |
| right_elbow | right_shoulder | right_elbow | right_wrist |
| left_elbow | left_shoulder | left_elbow | left_wrist |
| right_hip | left_hip | right_hip | right_knee |
| left_hip | right_hip | left_hip | left_knee |

# Fig. 10

501 — Obtain reference performers recordings

502 — Generate body points

503 — Determine reference landmark angles

504 — Training a machine learning model according to reference landmark angles for poses

505 — Obtain recording from user

506 — Generate body points

507 — Determine user landmark angles

508 — Evaluate user frame data to indicate which pose is represented in the frame

509 — Read probability of a pose

Next frame

Enough frames processed

510 — Repetition complete?

511 — Update number of repetitions

Next frame

**Fig. 11**

<u>601</u>

Register user

<u>602</u>

Collect user profile data

<u>603</u>

Collect training data

<u>604</u>

Perform training and evaluate performance

<u>605</u>

Compare current training with history of trainings

<u>606</u>

Determine exercises corresponding to user's state

<u>607</u>

Adapt training plan

<u>608</u>

Accept adaptation of training plan by supervisor

**Fig. 12**

**Fig. 13**

| | Europäisches Patentamt European Patent Office Office européen des brevets | **EUROPEAN SEARCH REPORT** | Application Number EP 23 46 1673 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/008413 A1 (ASIKAINEN SAMI [CA] ET AL) 14 January 2021 (2021-01-14) * paragraph [0002] * * paragraph [0006] - paragraph [0008] * * paragraph [0040] - paragraph [0043] * * paragraph [0046] * * paragraph [0070] - paragraph [0073] * * paragraph [0078] * * claims 1-10 * * Facsimile page 2 to page 3 * * Facsimile page 6 * * paragraph [0081] - paragraph [0083] * * paragraph [0093] - paragraph [0094] * ----- | 1-10 | INV. G16H20/30 G16H50/30 ADD. G16H30/40 G16H40/67 G16H50/20 |
| X | US 2022/079510 A1 (ROBILLARD JEAN [US] ET AL) 17 March 2022 (2022-03-17) * claims 1-8 * * Facsimile page 4 * * paragraph [0017] - paragraph [0033] * * paragraph [0041] - paragraph [0045] * * paragraph [0052] - paragraph [0070] * ----- | 1-10 | |

TECHNICAL FIELDS SEARCHED (IPC)

G16H

The present search report has been drawn up for all claims

| Place of search | Date of completion of the search | Examiner |
|---|---|---|
| Munich | 11 March 2024 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 46 1673**

This annex lists the patent family members relating to the patent documents cited in the above-mentioned European search report.
The members are as contained in the European Patent Office EDP file on
The European Patent Office is in no way liable for these particulars which are merely given for the purpose of information.

**11-03-2024**

| Patent document cited in search report | | Publication date | Patent family member(s) | | Publication date |
|---|---|---|---|---|---|
| US 2021008413 | A1 | 14-01-2021 | CA | 3146658 A1 | 14-01-2021 |
| | | | EP | 3996822 A1 | 18-05-2022 |
| | | | GB | 2600289 A | 27-04-2022 |
| | | | US | 2021008413 A1 | 14-01-2021 |
| | | | WO | 2021007581 A1 | 14-01-2021 |
| US 2022079510 | A1 | 17-03-2022 | AU | 2021342157 A1 | 20-04-2023 |
| | | | CA | 3192004 A1 | 17-03-2022 |
| | | | EP | 4210573 A1 | 19-07-2023 |
| | | | US | 2022079510 A1 | 17-03-2022 |
| | | | WO | 2022056271 A1 | 17-03-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LUNDBERG, SCOTT M.** ; **SU-IN LEE**. A unified approach to interpreting model predictions. *Advances in neural information processing systems*, 2017, vol. 30

- **BIECEK, PRZEMYSLAW** ; **TOMASZ BURZYKOWSKI**. Explanatory model analysis: explore, explain, and examine predictive models. CRC Press, 2021